# EUROPEAN PATENT APPLICATION

(11) **EP 3 643 361 A1**
(43) Date of publication of application: **29.04.2020**
(21) Application number: 18202119.6
(22) Date of filing: 23.10.2018
(51) Int. Cl.: A61Q 5/06, A61K 8/04, A61K 8/49, A61K 8/81

(54) **SHINE ENHANCING SPRAY COMPOSITION**

(71) Applicant: Kao Germany GmbH, 64297 Darmstadt (DE)
(72) Inventor: CAJAN, Christine, 64297 Darmstadt Hessen (DE); SCHMID, Sabine, 64297 Darmstadt (DE); UELLNER, Yuqi, 64297 Darmstadt (DE)

(57) **Abstract**

The present invention relates to a substantially anhydrous shine enhancing spray composition for keratin fibers, especially for human hair, comprising, in a cosmetically acceptable medium, one or more hair setting film forming polymers and a pyrrolidone carboxylic acid ester.

## Description

The present invention relates to a substantially anhydrous shine enhancing spray composition for keratin fibers, especially for human hair, comprising, in a cosmetically acceptable medium, one or more hair setting film forming polymers and a pyrrolidone carboxylic acid ester.

Substantially anhydrous compositions applied by spraying onto keratin fibers and especially hair have been used for setting said fibers to a certain shape for many years. They are based on a non-aqueous solvent and comprising at least one hair setting /film forming polymer. In order to realize certain flexibility to the formed film on the keratin fiber surface, usually surfactants, polyols, oils especially silicone oils are added. However, addition of these substances influences performance of the composition and causes reducing of the setting effect. Generally, it is known that the sprays used for setting hair do take away some of the natural hair shine so that the hair appears less shiny, and has therefore dull appearance. There is a great need for products providing shine and hair setting to keep the hair in desired shape and hold in the shape for a considerable period of time.

The present invention starts from the above known problems with the performance of the sprays, and aims at providing a spray composition which does not affect hair setting negatively and additionally enhances hair shine.

Pyrrolidone carboxylic acid and its esters are known in hair conditioners and being used for improving several hair properties. EP 2090291 discloses aqueous hair conditioning compositions comprising PCA and/or its salts, one or more PCA ester and a hair conditioning compound selected from quaternary ammonium polymers and surfactants. The document does not address any substantially anhydrous compositions.

The inventors of the present invention have unexpectedly found out that a substantially anhydrous composition comprising one or more pyrrolidone carboxylic acid ester and one or more hair setting film forming polymers does provide good hair setting and additionally provides hair additional shine. It is usually the case that the compounds added into to the substantially anhydrous compositions take away part of hair setting effect and do result in less shiny hair. The inventors of the present invention have observed the opposite effects when the substantially anhydrous hair setting composition is added pyrrolidone carboxylic acid ester.

Accordingly, the first object of the present invention is a substantially anhydrous composition for keratin fibers, especially for human hair, comprising, in a cosmetically acceptable substantially anhydrous medium, one or more hair setting film forming polymers and one or more pyrrolidone carboxylic acid ester according to general structure 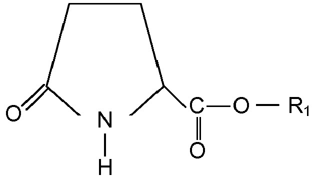 wherein R₁ is a straight or branched, saturated or unsaturated alkyl chain with 1 to 22 C atoms, phenyl, phenyl alkyl with alkyl chain length of 1 to 4 C atoms, phenyl alkoxy group with alkyl chain length of 1 to 4 C atoms and phenoxy alkyl group with alkyl chain length of 1 to 4 C atoms.

The second object of the present invention is the use of the compositions for providing keratin fibers, especially human hair setting, hold and shine.

Then third object is the method of setting and shine enhancing keratin fibers, especially human hair wherein the dry hair is applied the composition of the present invention, preferably by spraying.

The fourth object is a kit for keratin fibers, especially human hair comprising the composition of the present invention.

Within the meaning of the present invention, the term "substantially anhydrous" means that the composition comprises no added water and the water concentration present in the composition may not exceed 5% by weight, preferably 3% by weight, more preferably 1% by weight and most preferably 0.5% by weight, calculated to the total composition. The water may be present in the compositions because the raw materials used comprised water which may be bound water. In particularly preferred form the compositions are anhydrous.

Within the meaning of the present invention, the term "hair setting film forming polymers" means that the polymer has setting effect on the hair by forming film on the hair surface.

The composition of the present invention comprises at least one pyrrolidone carboxylic acid ester of the above formula. In the preferred form of the invention the R1 is alkyl with a chain length in the range of 8 to 20 C atoms, more preferably in the range of 12 to 18 C atoms and most preferably in the range of 14 to 18 C atoms.

Some examples to the suitable pyrrolidone carboxylic acid ester are with the alkyl chain of methyl, ethyl, n-propyl, isopropyl, n-butyl, iso butyl, n-hexyl, n-octyl, n-nonyl, n-decyl, ethylhexyl, methylhexyl, capryl, lauryl myristyl, cetyl, octyldodecyl, isostearyl, stearyl, arachidyl, behenyl, oleyl, linoleyl, benzyl, phenoxyethyl. Preferred are n-octyl, n-nonyl, n-decyl, ethylhexyl, methylhexyl, capryl, lauryl myristyl, cetyl, octyldodecyl, stearyl, isostearyl, arachidyl, behenyl, oleyl and linoleyl. More preferred are lauryl myristyl, cetyl, octyldodecyl, stearyl, isostearyl, arachidyl, behenyl, oleyl, and linoleyl. Particularly preferred pyrrolidon carboxylic acid ester is octyldodecyl pyrrolidone carboxylic acid which is available under the trade name Protelan ODPC from Zschimmer & Schwarz.

One or more pyrrolidone carboxylic acid esters is (are) comprised in the compositions of the present invention at a total concentration in the range of 0.01 to 15%, preferably 0.1 to 10%, more preferably 0.25 to 7.5% and most preferably 0.5 to 5% by weight, calculated to the total composition, in order to provide the desired effects.

The composition comprises one or more hair setting film forming polymers, preferably selected from anionic, cationic, non-ionic and amphoteric ones.

Non-limiting suitable non-ionic polymer is first of all vinylpyrrolidon polymers either homopolymers or copolymers with, especially, vinylacetate. Those are known with the trade name "Luviskol" as homopolymers Luviskol K 30, K 60 or K 90 as well copolymers Luviskol VA 55, VA 64, Plus from BASF AG.

Natural non-ionic polymers are as well suitable for the composition of the present invention. Those are such as starch, cellulose, chitosan, guar gum and their derivatives.

As amphoteric polymers which can be used alone or in mixture with at least one additional non-ionic polymer, reference is here made in particular to copolymers of N-octyl acrylamide, (meth)acrylic acid and tert.-butyl aminoethyl methacrylate of the type "Amphomer®"; copolymers from methacryloyl ethyl betaine and alkyl methacrylates of the type "Yukaformer®", e.g.. the butyl methacrylate copolymer "Yukaformer® Am75"; copolymers from monomers containing carboxyl groups and sulfonic groups, e.g.. (meth)acrylic acid and itaconic acid, with monomers such as mono- or dialkyl aminoalkyl (meth)acrylates or mono- or dialkyl aminoalkyl (meth)-acrylamides containing basic groups, in particular amino groups; copolymers from N-octyl acrylamide, methyl methacrylate, hydroxypropyl methacrylate, N-tert.-butyl aminoethyl methacrylate and acrylic acid, as well as the copolymers known from USA 3,927,199.

Non-limiting suitable anionic polymers alone or in combination with non-ionic polymers are vinyl alkyl ether, in particular methyl vinyl ether/maleic acid copolymers, obtained by hydrolysis of vinyl ether/maleic anhydride copolymers, distributed under the trade name "Gantrez® AN or ES". These polymers may also be partly esterified, as for example, "Gantrez® ES 225" or "ES 435", the ethyl ester of an ethyl vinyl ether/maleic acid copolymer, or the butyl or isobutyl ester thereof.

Further useful anionic polymers are in particular vinyl acetate/crotonic acid or vinyl acetate/vinyl neodecanoate/crotonic acid copolymers of the type "Resyn®"; sodium acrylate/vinyl alcohol copolymers of the type "Hydagen® F", sodium polystyrene sulfonate, e.g.. "Flexan® 130"; ethyl acrylate/acrylic acid/N-tert.-butyl acrylamide copolymers of the type "Ultrahold®"; vinyl pyrrolidone/vinyl acetate/itaconic acid copolymers, acrylic acid/acrylamide copolymers or the sodium salts thereof of the type "Reten®"; etc.

Further suitable anionic polymers are Acrylate copolymers available under trade name Salcare SC 81, PEG/PPG 25/25 dimethicone / acrylate copolymer available under trade name Luvigel Silk from BASF, Acrylates/t-butylacrylamide copolymer available under trade name Ultrahold Strong, Advantage LC-E which is vinylcaprolactam/PVP/dimethylaminoethylmethacrylate copolymer and VA/crotonates copolymer available under trade name Luviset CA 66.

Further suitable and preferred anionic polymer is acrylates/octylacrylamide copolymer available under the trade name Amphomer.

The anionic polymers are preferably neutralized either partially or completely, 100%, with an alkalizing agent. The suitable alkalizing agents are alkanolamines and especially preferred is aminomethlypropanol.

Non-limiting suitable cationic polymers are those known with their CTFA category name Polyquaternium. Typical examples of those are Polyquaternium-6, Polyquaternium-7, Polyquaternium-10, Polyquaternium-11, Polyquaternium-16, Polyquaternium-22 and Polyquaternium- 28, Polyquaternium-30, Polyquaternium-37, Polyquaternium-36, Polyquaternium-46, Polyquaternium-24, Polyquaternium-67, and Polyquaternium-72.

In this context, reference is also made to the cationic polymers disclosed in DE 25 21 960, 28 11 010, 30 44 738 and 32 17 059, as well as to the products described in EP-A 337 354 on pages 3 to 7, It is also possible to use mixtures of various cationic polymers.

The cationic polymers also include the quaternized products of graft polymers from organopolysiloxanes and polyethyl oxazolines described in EP-A 524 612 and EP-A 640 643. Among these especially preferred is the compound know with the INCI name Polysilicone-9.

The compositions comprise one or more hair setting film forming polymers at a total concentration in the range of 0.1 to 25% by weight, preferably 0.5 to 20% by weight, more preferably 1.0 to 15% by weight and most preferably 2 to 15% by weight, in particular 3 to 15% by weight, calculated to the total of the composition.

Substantially anhydrous composition of the present invention comprises organic solvents as the cosmetically acceptable medium. The compositions comprise one or more organic solvents. Suitable ones are ethanol, propanol, isopropanol, isopentane, n-pentane, n-hexane, dimethoxymethane, benzyl alcohol, benzyloxyethanol, alkylene carbonates such as ethylene carbonate and propylene carbonate, phenoxyethanol, butanol, isobutanol, cyclohexane, cyclohexanol, 1-phenylethylalcohol, 2-phenylethylalcohol, o-methoxyphenol. The preferred organic solvents are ethanol, isopropanol and propanol. The most preferred organic solvent is ethanol. The total concentration of one or more organic solvents is in the range of 30 to 95%, preferably 40 to 90%, more preferably 45 to 85%, most preferably 50 to 80% and in particular 60 to 75% by weight calculated to the total composition.

The composition of the present invention may be applied onto hair by spraying from a mechanical spraying device as well as form an aerosol device. In case an aerosol type of styling product is designed, the composition comprises additionally one or more propellants. Suitable propellants are lower alkanes such as n-butane, i-butane, propane, butane or their mixtures, as well as dimethylether (DME) either alone or in mixture with lower alkanes. Further suitable propellants are fluorinated hydrocarbons such as 1,1-difluoro ethane (Hydrofluorocarbon 152a) or tetrafluoroethane or their mixtures with each other, carbon dioxide and nitogen or their mixtures with the above mentioned propellants. Preferred are lower alkanes especially propane, butane, n-butane, i-butane, and their mixtures, 1,1-difluoro ethane (Hydrofluorocarbon 152a) and their mixture with DME at a alkane to DME weight ratio 10:1 to 1:10.

One or more propellants are comprised in the compositions at a concentration in the range of 10 to 80%, preferably 20 to 70%, more preferably 30 to 65% and most preferably 40 to 60% by weight, calculated to the total composition.

It should be noted all of the concentrations given in this specification for the components are calculated to the composition which may be non-aerosol composition without propellant or an aerosol composition with propellant.

In an embodiment of the present invention, compositions additionally comprise at least one polyol. The term polyol refers to a compound carrying 2 or more hydroxyl groups in its molecule.

Non-limiting suitable examples to the polyols preferred within the scope of the present invention are ethylene glycol and its homo polymers with varying molecular weight known with their INCI names such as diethyleneglycol, triethylene glycol and PEG with 4 to 800 ethylene glycol units, propylene glycol and its homo polymers with varying molecular weight known with their INCI names such as dipropylene glycol, tripropylene glycol and PPG with 4 to 50 propylene glycol units and glycerin and its homo polymers known with their INCI names diglycerin, triglycerin and polyglycerin with 4 to 40 gylcerin units. Any of the homopolymers mentione here can be liquid, semisolid, or solid. In the preferred from of the invention suitable polyols are the ones either liquid or semiliquid at ambient temperature. The most preferred are liquid ones at ambient temperature such as propylenegylcol, dipropylene glycol, PPG-9, diethylene glycol, triethylene glycol and glycerin.

The total concentration of one or more polyols in the composition is in the range of 0.01 to 2.5%, preferably 0.05 to 2%, more preferably 0.1 to 1.5% and most preferably 0.1 to 1% by weight, calculated to the total composition.

The composition may comprise one or more synthetic or natural oil (s). In principal, any oil allowed for cosmetic use is suitable for the compositions of the present invention.

Suitable oils are those of synthetic and of natural ones. Natural oils are in principal any triglyceride suitable for cosmetic use. Non-limiting examples are avocado oil, coconut oil, palm oil, sesame oil, peanut oil, whale oil, sunflower oil, almond oil, peach kernel oil, wheat germ oil, macadamia nut oil, night primrose oil, jojoba oil, castor oil, or also olive oil, soya oil, and the derivatives thereof. Mineral oils such as paraffin oil and petrolatum are suitably contained within the scope of the present invention, It should as well be noted that compositions of the present invention can contain mixture of one or more natural oils and mineral oil.

Further, suitable synthetic oil components are in particular fatty alcohol fatty acid esters such as isopropyl myristate, palmitate, stearate and isostearate, oleyl oleate, isocetyl stearate, hexyl laurate, dibutyl adipate, dioctyl adipate, myristyl myristate, oleyl erucate, polyethylene glycol and polyglyceryl fatty acid esters, cetyl palmitate, etc.

Synthetic ones are those of silicone oils. Here again any silicone oil either volatile and/or non-volatile is suitable for the compositions of the present invention. Preferred silicone oils are non-volatile silicone oils known with their INCI name as dimethicone and dimethiconol. Volatile silicone oils such as cyclomethicones may be used in combination with non-volatile silicones and/or other wax and/or oils mentioned above. Commercially, they are available from various companies for example Dow Corning with the known DC series, Wacker Chemie and Toray silicones. All commercially available non volatile silicones are suitable in the compositions of the present invention. Examples to those are DC 200 series, DC1401, DC 1403, DC 1501 and DC 1503. Furthermore, aminated silicones such as amodimethicone and arylated silicones comprising at least one aryl group in its molecule such as phenyl methicone, phenyl trimethicone, diphenyl dimethicone, diphenylsiloxy phenyl trimethicone, tetramethyl tetraphenyl trisiloxane, triphenyl trimethicone, tetramethyl tetraphenyl trisiloxane and trimethyl pentaphenyl trisiloxane can be advantageously comprised in the compositions of the present invention.

The total concentration of one or more oils is in the range of 0.01 to 1% by weight calculated to the total composition.

Additionally natural plant extracts can as well form part of the compositions of the present invention. Those are incorporated usually in an amount of 0.01 % to 0.5 %, by weight, calculated as dry residue thereof to the total composition. Suitable aqueous (e.g. steam-distilled) alcoholic or hydro-alcoholic plant extracts known **per se** are in particular extracts from leaves, fruits, blossoms, roots, rinds or stems of aloe, pineapple, artichoke, arnica, avocado, valerian, bamboo, henbane, birch, stinging nettle, echinacea, ivy, wild angelica, gentian, ferns, pine needles, silver weed, ginseng, broom, oat, rose hip, hamamelis, hay flowers, elderberry, hop, coltsfoot, currants, chamomile, carrots, chestnuts, clover, burr root, coconut, cornflower, lime blossom, lily of the valley, marine algae, balm, mistletoe, passion flower, ratanhia, marigold, rosemary, horse chestnut, pink hawthorn, sage, horsetail, yarrow, primrose, nettle, thyme, walnut, wine leaves, white hawthorn, etc.

Suitable trade products are, for example, the various "Extrapon®" products, "Herbasol^{R}", "Sedaplant^{R}" and "Hexaplant^{R}". Extracts and the preparation thereof are also described in "Hagers Handbuch der pharmazeutischen Praxis". 4th Ed..

Compositions of the present invention may comprise one or more UV filters either for stabilization of the product colour and/or for protection of hair from environmental influences such as loss of elasticity, loss of hair colour (bleaching effect of sun light). Suitable UV-absorbing substance are Polysilicone-15, 4-Aminobenzoic acid and the esters and salts thereof, 2-phenyl benzimidazole-5-sulfonic acid and the alkali and amine salts thereof, 4-dimethyl aminobenzoic acid and the esters and salts thereof, cinnamic acid and the esters and salts thereof, 4-methoxycinnamic acid and the esters and salts thereof, salicylic acid and the esters and salts thereof, 2,4-dihydroxybenzophenone, 2,2',4,4'-tetrahydroxy- benzophenone, 2-hydroxy-4-methoxybenzophenone and its 5-sulfonic acid or the sodium salt thereof, 2,2'-dihydroxy-4,4'-dimethoxybenzophenone, 2-hydroxy-5-chlorobenzophenone, 2,2'-dihydroxy-4-methoxybenzophenone, 2,2'-dihydroxy-4,4'-dimethoxy-5,5'-disulfobenzophenone or the sodium salt thereof, 2-hydroxy-4-octyloxybenzophenone, 2-hydroxy-4-methoxy-4'-methylbenzophenone, 3-benzyl-idenecampher, 3-(4'-sulfo)-benzyl-idenebornane-2-one and the salts thereof and/or 3-(4'-methyl benzylidene)-DL-campher, and/or polysiliocne-15.

The suitable amount of the UV-absorber ranges from about 0.01 % to 1% by weight, calculated to the total composition. Attention should be paid to the stability and solubility especially when using UV filter as salts, e.g. anionic UV filter salts.

The composition may comprises pyrrolidone carboxylic acid and/or its salts. In principal any pyrrolidone carboxylic acid salt is suitable within the meaning of the present invention. Suitable non-limiting examples are aluminium, calcium, copper, magnesium, potassium, sodium and zinc salts of pyrrolidone carboxylic acid. Preferred are aluminium, calcium, magnesium, potassium and sodium salts and more preferred are sodium and potassium salts. The most preferred is sodium salt.

The total concentration of pyrrolidone carboxylic acid and/or its salts is in the range of 0.01 to 10%, preferably 0.05 to 7.5%, more preferably 0.1 to 5% and most preferably 0.2 to 3% by weight calculated to the total composition.

The compositions may comprise further compounds which are customarily found in such compositions such as fragrance, neutralizing agents, solubilizing agents, preservatives, etc.

The following examples are to illustrate the invention but not to limit it.

### Example 1

The following compositions were prepared.

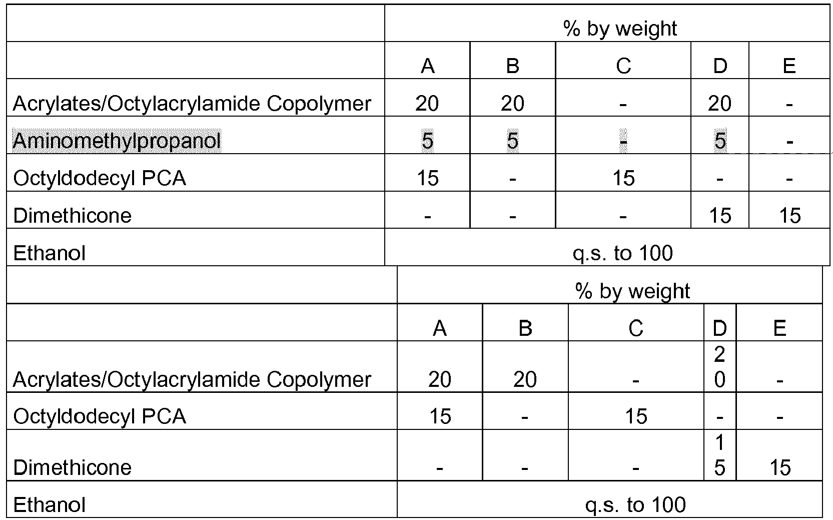

The composition A is according to the present invention and the other compositions B to E are comparative examples.

The compositions were filled into an aerosol can as follows:

| | % by weight |
|---|---|
| The solution of Example 1 | 20.0 |
| n-pentane | 20.0 |
| Hydrofluorocarbon 152a | 60.0 |

For this purpose, the hair streak weighing approximately 2 g was washed first with a commercially available cleansing composition and dried with a hair dryer. The dry hair was applied the aerosol compositions above onto hair at a composition to hair weight ratio of 1:2 The hair steaks were left to dry in the air and the bending force and the shine were measured as disclosed below.

The bending force of the hair was measured with a zwick machine and expressed as the force (in milliNewton - mN). The machine measured the force needed to push down the streak between two rods. The machine is commercially available from Zwick/Roell Company and includes a manual. It was used according to its manual.

The shine measurement was carried out with the Samba Hair System from Bosa Nova Technologies via polarized illumination. The hair tress was illuminated and the specular reflection was measured. This gives information about the gloss of the hair.

The shine factor was calculated with the Reich Robins method.

The following results were obtained

| Composition | Bending force (mN) | Shine Factor |
|---|---|---|
| A | 0.62 | 30.45 |
| B | 0.48 | 11.32 |
| C | 0.14 | 17.50 |
| D | 0.29 | 22.49 |
| E | 0.09 | 15.60 |

It should be noted, for the bending force, the higher the number, the higher the hold of the style. For the shine factor, in the same way, the higher the number, the higher the shine.

From the above results, it is beyond any doubt that the composition according to the present invention has the highest score in bending force, indicating clearly the best hold and the highest score in shine factor indicating the best shine. It must be pointed out that silicones are known to enhance shine of hair; however, they even failed to enhance shine as good as the inventive composition. Furthermore, the bending force of the polymer film is reduced dramatically in the presence of dimethicone, however, presence of PCA ester increased the bending force showing clearly the better hold.

The results obtained with the individual compounds do not provide any indication to predict the combined effect.

### Example 2

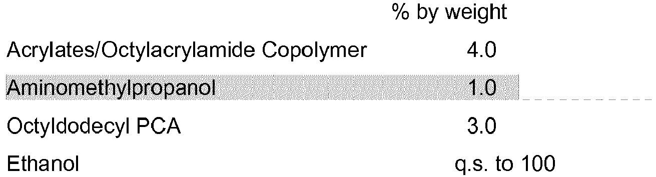

The above composition was applied in the same way as in Example 1 using a mechanical spraying device (pump spray) and the bending force and shine factor measurements showed and confirmed the same effects.

The following examples are within the scope of the present invention and showed the similar effects on the hair.

### Example 3

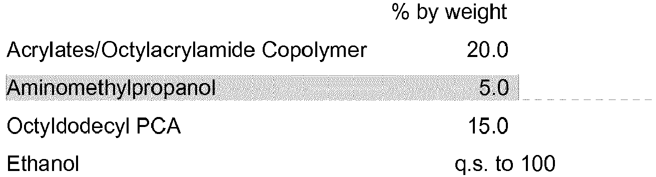

The composition was filled into an aerosol can as follows:

| | % by weight |
|---|---|
| The solution of Example 3 | 20.0 |
| n-pentane | 20.0 |
| Dimethylether (DME) | 60.0 |

### Example 4

| | % by weight |
|---|---|
| VP/ Vinylcaprolactam/DMAPA Acrylates Copolymer | 20.0 |
| Octyldodecyl PCA | 15.0 |
| Ethanol | q.s. to 100 |

The composition was filled into an aerosol can as follows:

| | % by weight |
|---|---|
| The solution of Example 4 | 20.0 |
| n-pentane | 20.0 |
| Hydrofluorocarbon 152a | 60.0 |

The same composition was also filled into a second aerosol can as follows:

| | % by weight |
|---|---|
| The solution of Example 4 | 30.0 |
| Propane/butane | 40.0 |
| DME | 30.0 |

### Example 5

| | % by weight |
|---|---|
| VP/ VA Copolymer | 20.0 |
| Octyldodecyl PCA | 15.0 |
| Ethanol | q.s. to 100 |

The composition was filled into an aerosol can as follows:

| | % by weight |
|---|---|
| The solution of Example 5 | 20.0 |
| n-pentane | 20.0 |
| Hydrofluorocarbon 152a | 60.0 |

The same composition was also filled into a second aerosol can as follows:

| | % by weight |
|---|---|
| The solution of Example 5 | 30.0 |
| Propane/butane | 40.0 |
| DME | 30.0 |

### Example 6

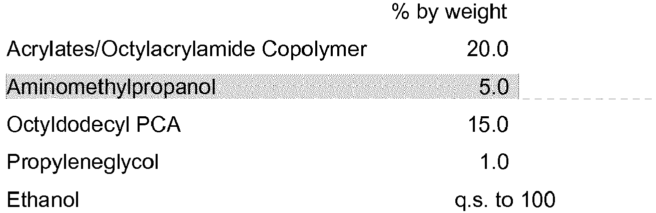

### Example 7

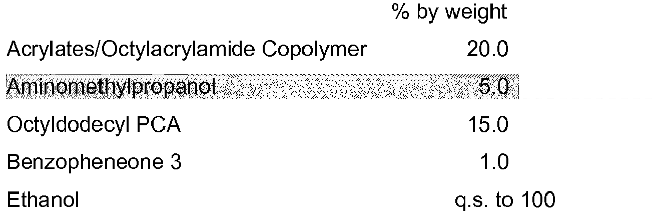

### Example 8

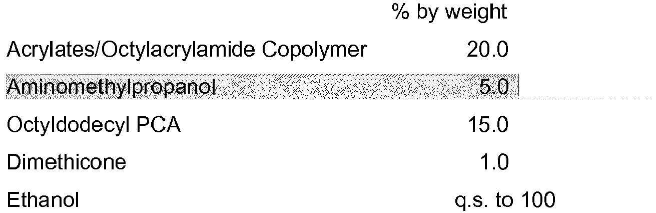

### Example 9

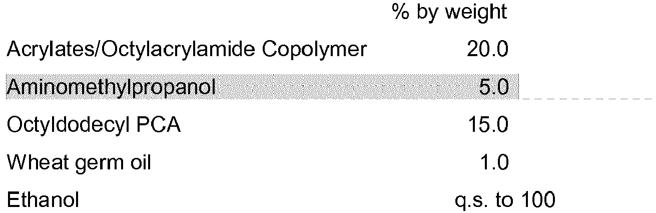

### Example 10

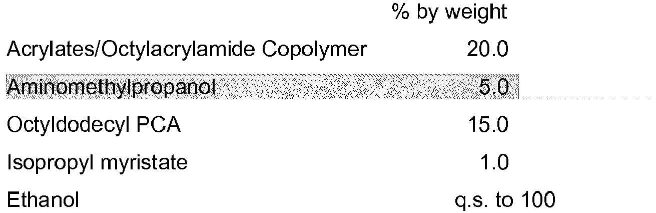

### Example 11

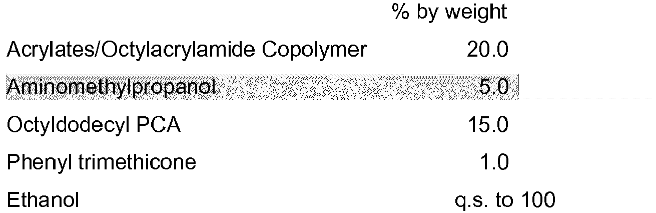

### Example 12

| | % by weight |
|---|---|
| VP/VA Copolymer | 20.0 |
| Octyldodecyl PCA | 15.0 |
| Propyleneglycol | 1.0 |
| Ethanol | q.s. to 100 |

### Example 13

| | % by weight |
|---|---|
| VP/VA Copolymer | 20.0 |
| Octyldodecyl PCA | 15.0 |
| Benzopheneone 3 | 1.0 |
| Ethanol | q.s. to 100 |

### Example 14

| | % by weight |
|---|---|
| VP/VA Copolymer | 20.0 |
| Octyldodecyl PCA | 15.0 |
| Dimethicone | 1.0 |
| Ethanol | q.s. to 100 |

### Example 16

| | % by weight |
|---|---|
| VP/VA Copolymer | 20.0 |
| Octyldodecyl PCA | 15.0 |
| Wheat germ oil | 1.0 |
| Ethanol | q.s. to 100 |

### Example 17

| | % by weight |
|---|---|
| VP/VA Copolymer | 20.0 |
| Octyldodecyl PCA | 15.0 |
| Isopropyl myristate | 1.0 |
| Ethanol | q.s. to 100 |

### Example 18

| | % by weight |
|---|---|
| VP/VA Copolymer | 20.0 |
| Octyldodecyl PCA | 15.0 |
| Phenyl trimethicone | 1.0 |
| Ethanol | q.s. to 100 |

### Example 19

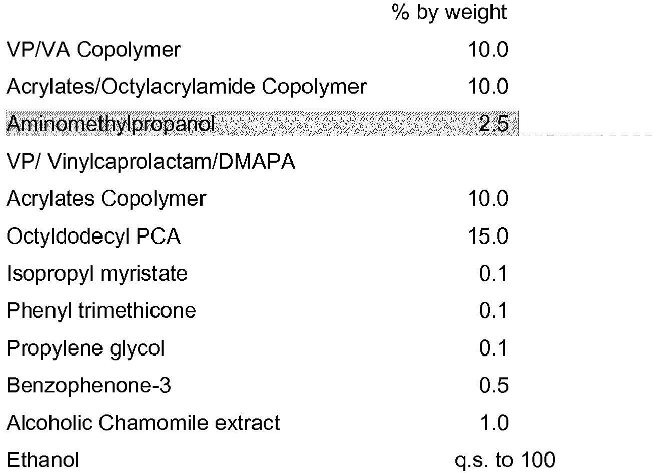

The Examples 6 to 19 were used as follows:

### Non-aerosol compositions:

| | % by weight |
|---|---|
| The solution of Examples | 20 |
| Ethanol | 80 |

The non-aerosol compositions were applied onto hair using a mechanical spraying device (pump spray).

### Aerosol compositions

### Option 1:

| | % by weight |
|---|---|
| The solution of Examples | 20.0 |
| n-pentane | 20.0 |
| Hydrofluorocarbon 152a | 60.0 |

### Option 2:

| | % by weight |
|---|---|
| The solution of Examples | 30.0 |
| Hydrofluorocarbon 152a | 70.0 |

### Option 3:

| | % by weight |
|---|---|
| The solution of Examples | 20.0 |
| n-pentane | 20.0 |
| DME | 60.0 |

### Option 4:

| | % by weight |
|---|---|
| The solution of Example | 30.0 |
| Propane/butane | 40.0 |
| DME | 30.0 |

## Claims

1. A substantially anhydrous composition for keratin fibers, especially for human hair, **characterized in that** it comprises, in a cosmetically acceptable substantially anhydrous medium, one or more hair setting film forming polymers and one or more pyrrolidone carboxylic acid ester according to general structure 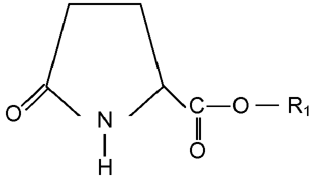 wherein R₁ is a straight or branched, saturated or unsaturated alkyl chain with 1 to 22 C atoms, phenyl, phenyl alkyl with alkyl chain length of 1 to 4 C atoms, phenyl alkoxy group with alkyl chain length of 1 to 4 C atoms and phenoxy alkyl group with alkyl chain length of 1 to 4 C atoms.

2. The composition according to claim 1 **characterized in that** hair setting film forming polymers is selected from anionic, non-ionic, cationic and amphoteric ones and their mixtures.

3. The composition according to claims 1 and 2 **characterized in that** pyrrolidone carboxylic acid ester is octyldodecyl pyrrolidone carboxylic acid.

4. The composition according to any of the preceding claims **characterized in that** it comprises one or more hair setting film forming polymers at a total concentration in the range of 0.1 to 25% by weight, preferably 0.5 to 20% by weight, more preferably 1.0 to 15% by weight and most preferably 2 to 15% by weight, in particular 3 to 15% by weight, calculated to the total of the composition.

5. The composition according to any of the preceding claims **characterized in that** it comprises one or more pyrrolidone carboxylic acid ester at a total concentration in the range of 0.01 to 15%, preferably 0.1 to 10%, more preferably 0.25 to 7.5% and most preferably 0.5 to 5% by weight, calculated to the total composition.

6. The composition according to any of the preceding claims **characterized in that** it comprises one or more hair setting film forming anionic polymers preferably comprised as partially or completely (100%) neutralized with an alkalizing agent selected from alkanolamines, preferably it is aminomethylpropanol.

7. The composition according to any of the preceding claims **characterized in that** it comprises one or more organic solvents, preferably selected from ethanol, isopropanol and propanol, more preferably it is ethanol.

8. The composition according to claim 7 **characterized in that** it comprises one or more organic solvents at a concentration in the range of 30 to 95%, preferably 40 to 90%, more preferably 45 to 85%, most preferably 50 to 80% and in particular 60 to 75% by weight calculated to the total composition.

9. The composition according to any of the preceding claims **characterized in that** it comprises one or more propellants, preferably at a concentration in the range of 10 to 80%, more preferably 20 to 70%, even more preferably 30 to 65% and most preferably 40 to 60% by weight, calculated to the total composition.

10. The composition according to any of the preceding claims **characterized in that** one or more propellants is (are) selected from n-butane, i-butane, propane, butane dimethylether (DME), 1,1-difluoro ethane (Hydrofluorocarbon 152a) and tetrafluoroethane.

11. The composition according to any of the preceding claims **characterized in that** it comprises water less than 5% by weight, preferably less than 3% by weight, more preferably less than 1% by weight and most preferably less than 0.5% by weight, calculated to the total composition, in particular it is anhydrous.

12. The composition according to any of the preceding claims **characterized in that** it comprises one or more of the following ingredients
- One or more polyols
- One or more UV filter
- One or more oil selected from natural and synthetic oils, and
- One or more natural plant extracts.

13. The use of the composition according to any of ten preceding claims for providing keratin fibers, especially human hair, setting, hold and shine.

14. The method of setting and shine enhancing and providing hold to keratin fibers, especially human hair wherein the dry hair is applied the composition according to claims 1 to 12, preferably by spraying.

15. A kit for keratin fibers, especially human hair comprising the composition according to claims 1 to 12.
